(19) Europäisches Patentamt
European Patent Office
Office européen des brevets

(11) **EP 4 101 468 A1**

(12) **EUROPEAN PATENT APPLICATION**
published in accordance with Art. 153(4) EPC

(43) Date of publication:
**14.12.2022 Bulletin 2022/50**

(21) Application number: **21750742.5**

(22) Date of filing: **04.02.2021**

(51) International Patent Classification (IPC):
***A61K 45/06*** (2006.01) ***A61P 35/00*** (2006.01)
***A61P 35/02*** (2006.01) ***A61P 43/00*** (2006.01)
***A61K 31/404*** (2006.01) ***A61K 31/437*** (2006.01)
***A61K 31/519*** (2006.01) ***A61K 31/53*** (2006.01)
***A61K 31/5377*** (2006.01)

(52) Cooperative Patent Classification (CPC):
**A61K 31/404; A61K 31/437; A61K 31/519; A61K 31/53; A61K 31/5377; A61K 45/06; A61P 35/00; A61P 35/02; A61P 43/00**

(86) International application number:
**PCT/JP2021/004083**

(87) International publication number:
**WO 2021/157650 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.02.2020 JP 2020018099**

(71) Applicant: **Carna Biosciences, Inc.**
**Kobe-shi, Hyogo 650-0047 (JP)**

(72) Inventors:
- **SAWA, Masaaki**
  **Kobe-shi, Hyogo 650-0047 (JP)**
- **NISHIOKA, Yu**
  **Kobe-shi, Hyogo 650-0047 (JP)**
- **ENDO, Hiroko**
  **Kobe-shi, Hyogo 650-0047 (JP)**

(74) Representative: **Kador & Partner PartG mbB**
**Corneliusstraße 15**
**80469 München (DE)**

(54) **ANTICANCER AGENT COMPOSITION**

(57) This invention provides with a novel means to treat a cancer, in which reversible BTK inhibitor is combined with a BCL-2 inhibitor. Specifically an anticancer agent composition in which a BTK inhibitor below;

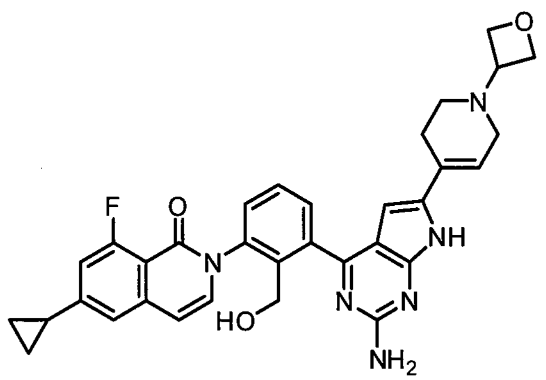

is combined with venetoclax.

Figure 1

| Venetoclax (nM) \ (I −A) (nM) | 300 | 90 | 30 | 9 | 3 | 0.9 | 0.3 | 0 |
|---|---|---|---|---|---|---|---|---|
| 90 | 99 | 100 | 100 | 98 | 95 | 95 | 93 | 92 |
| 30 | 98 | 98 | 96 | 90 | 82 | 77 | 74 | 74 |
| 9 | 98 | 96 | 89 | 73 | 60 | 51 | 53 | 50 |
| 3 | 96 | 93 | 79 | 56 | 40 | 32 | 31 | 25 |
| 0.9 | 95 | 91 | 68 | 43 | 25 | 20 | 15 | 11 |
| 0.3 | 95 | 90 | 66 | 39 | 19 | 12 | 11 | 5 |
| 0.09 | 93 | 89 | 61 | 26 | 9 | 6 | 3 | 1 |
| 0 | 93 | 87 | 63 | 22 | 4 | -3 | -11 | 0 |

EP 4 101 468 A1

## Description

TECHNICAL FIELD

The present invention relates to an anticancer agent composition. More specifically, the present invention relates to an anticancer agent composition containing a combination of a reversible BTK inhibitor and a BCL-2 inhibitor.

BACKGROUND ART

Bruton's tyrosine kinase (BTK) is a member of the Tec family of non-receptor tyrosine kinases and is an important signaling enzyme expressed in all hematopoietic cell types except T lymphocytes and natural killer cells. BTK is an important regulator of survival, differentiation, proliferation, activation, and the like of B cells, and plays an important role in B cell signaling (Non Patent Documents 1 and 2). B-cell receptors (BCRs) on the cell surface transmit signals into the cell via BTK present downstream thereof. Thus, abnormal activation of the B cell signaling pathway is considered to promote proliferation and survival of cancer cells of B-cell lymphoma, chronic lymphocytic leukemia and the like (Non Patent Document 3).

BTK is also known to play an important role in signaling pathways of many other cells and is deemed to be involved in allergic diseases, autoimmune diseases and inflammatory diseases (Non Patent Document 1). For example, BTK plays an important role in signaling of high affinity IgE receptors (FcεRI) in mast cells, and BTK-deficient mast cells are known to have reduced degranulation and decreased production of proinflammatory cytokines (Non Patent Document 4).

It has also been suggested in experiments in BTK-deficient mice that BTK is involved in systemic lupus erythematosus (SLE) (Non Patent Document 5). Furthermore, BTK mutant mice exhibit resistance to the onset of collagen-induced arthritis (Non Patent Document 6). The irreversible BTK inhibitor drug ibrutinib is an anticancer agent used in the treatment of B-cell tumors. In recent years, it has been revealed that C481S mutation in BTK results in ibrutinib resistance during ibrutinib treatment (Non Patent Document 7). More recently, an isoform p65BTK has also been reported to be expressed downstream of RAS signals in solid cancers other than hematological cancers and to have a profound effect on proliferation in solid cancers such as colon cancer cells (Non Patent Document 8).

Thus, compounds having BTK inhibitory activity are considered to be useful for the treatment of diseases in which BTK signals are involved, such as cancer, B-cell lymphoma and chronic lymphocytic leukemia, and also useful for the treatment of solid cancers in which p65BTK is expressed. The compounds are also useful for the treatment of allergic diseases, autoimmune diseases, inflammatory diseases, and the like. Meanwhile, there is a need for reversible BTK inhibitor drugs that are effective for cancers that have mutations in BTK and are resistant to irreversible BTK inhibitors such as ibrutinib. In particular, oxoisoquinoline and triazine derivatives having reversible BTK inhibitory actions have been reported (see Patent Documents 1 and 2).

B-cell lymphoma 2 (BCL-2) is a member of the BCL-2 family that controls cell death. BCL-2 functions · mainly on mitochondrial membranes and negatively regulates apoptosis by positively and negatively modulating mitochondrial outer membrane permeability. Since BCL-2 is a molecule found through translocation frequently observed in follicular lymphoma, and has subsequently been reported to be activated in lymphoid B-cell tumors such as diffuse lymphoma (DLBCL) and chronic lymphocytic leukemia (CLL) as well as in multiple myeloma and T-cell tumors, molecules that inhibit BCL-2 are useful for the treatment of these cancers (Non Patent Document 9). Several BCL-2 inhibitors have been reported to date, and these BCL-2 inhibitors are known to inhibit the action of BCL-2 by selectively binding to BCL-2, induce potent apoptosis either alone or in combination with other therapeutic agents, and exhibit antitumor effects (Non Patent Document 10).

As mentioned above, BTK inhibitors have already been clinically used in anticancer therapy, but patients on whom the inhibitors had no effect have been reported and satisfactory therapeutic effects have not always been achieved (Non Patent Document 11).

In recent years, the combination of ibrutinib that is a BTK inhibitor and venetoclax that is a BCL-2 inhibitor has been reported to enhance antitumor effect in hematological tumors. Thus, the combination therapy of BTK inhibitor and Bcl-2 inhibitor is expected to be a new therapeutic method for cancer (Patent Document 3, Non Patent Document 12). However, the combination of a reversible BTK inhibitor and a BCL-2 inhibitor according to the present invention has not been disclosed at all, and anticancer actions by the combination of a reversible BTK inhibitor and a BCL-2 inhibitor according to the present invention have never been reported.

PRIOR ART DOCUMENTS

PATENT DOCUMENTS

[Patent Document 1] WO 2018/097234
[Patent Document 2] WO 2015/012149
[Patent Document 3] WO 2014/168975

NON PATENT DOCUMENTS

[Non Patent Document 1] Satterthwaite,A.B.and Witte,O.N., Immunol.Rev., 2000, 175, 120-127
[Non Patent Document 2] Kurosaki,T., Curr.Opin.Immunol., 2000, 12, 276-281
[Non Patent Document 3] Davis,R.E.,et al., Nature, 2010, 463, 88-92
[Non Patent Document 4] Ellmeier,W.,et al., FEBS J., 2011, 278, 1990-2000
[Non Patent Document 5] Halcomb,K.E., Mol.Immunol., 2008, 46(2), 233-241
[Non Patent Document 6] Jansson,L.and Holmdahl,R., Clin.Exp.Immunol., 1993, 94, 459-465
[Non Patent Document 7] Cheng,S., et al., Leukemia,2014,1-6
[Non Patent Document 8] Grassili.E.,et al., Oncogene, 2016, 35, 4368-4378
[Non Patent Document 9] Czabotar,P.,et al., Nature Rev. Mol. Cell Biol., 2014, 15, 49-63,
[Non Patent Document 10] Ashkenazi,A.,et al., Nature Rev. Drug Discov., 2017, 16, 273-284
[Non Patent Document 11] Wilson W.H.,et al., Nat.Med., 2015, 21(8), 922-926
[Non Patent Document 12] Kuo HP.,et al., Mol.Cancer Ther., 2017, 16(7), 1246-1256

SUMMARY OF INVENTION

PROBLEMS TO BE SOLVED BY THE INVENTIONS

An objective of the present invention is to provide with a composition comprising an anticancer agent to effectively induce cell death on a cancer cell by means of combining a reversible BTK inhibitor with a BCL-2 inhibitor.

MEANS OF SOLVING THE PROBLEMS

The present invention is related to a composition comprising an anticancer agent by combination of a reversible BTK inhibitor and a BCL-2 inhibitor. Specifically it is related to follows;

(1) A pharmaceutical composition for treating a cancer comprising a reversible BTK inhibitor and a BCL-2 inhibitor;
(2) A pharmaceutical composition described in (1), wherein the reversible BTK inhibitor is an oxoisoquinoline derivative of the formula (I)

[chem 1]

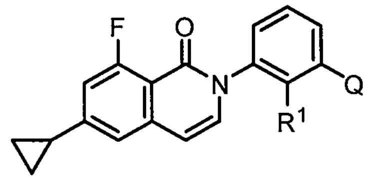

(I)

wherein $R^1$ is an optionally substituted lower alkyl, Q is a structure selected from the following structures (a), (b) and (c);

[chem 2]

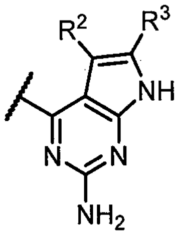 , 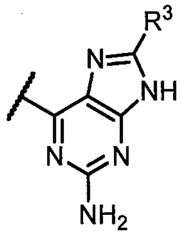 , 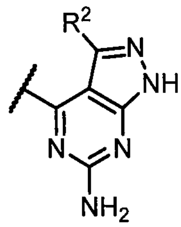

(a) (b) (c)

$R^2$ and $R^3$ are independently a hydrogen atom, an optionally substituted lower alkyl group, an optionally substituted cycloalkyl group, an optionally substituted aryl group, an optionally substituted heteroaryl group or an optionally substituted heterocyclic group,

or a pharmaceutically acceptable salt thereof;

(3) the pharmaceutical composition described in (2), wherein the reversible BTK inhibitor is an oxoisoquinoline derivative in which Q is a structure (a), and $R^1$ is a hydroxymethyl group,

(4) the pharmaceutical composition described in (1), wherein the reversible BTK inhibitor is an oxoisoquinoline derivative (Ia) shown below,

[chem 3]

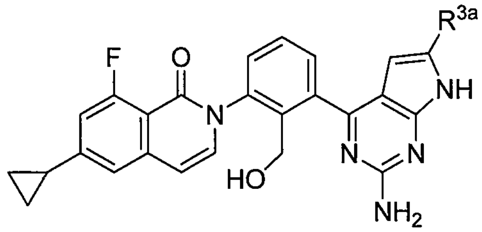

( Ia )

wherein $R^{3a}$ is an optionally substituted tetrahydropyridyl group,

or a pharmaceutically acceptable salt thereof;

(5) the pharmaceutical composition described in (4), wherein the reversible BTK inhibitor is an oxoisoquinoline derivative having a structure of Compound (I-A);

Compound (I-A): 2-(3-{2-amino-6-[1-(oxetan-3-yl)-1,2,3,6-tetrahydropyridin-4-yl]-7H-pyrrolo[2,3-d]pyrimidin-4-yl}-2-(hydroxymethyl)phenyl)-6-cyclopropyl-8-fluoroisoquinolin-1(2H)-one

[chem 4]

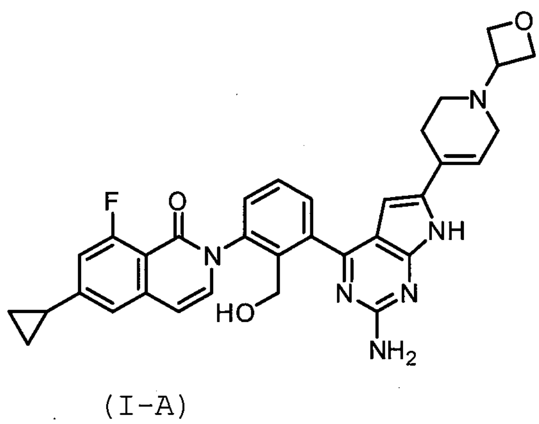

(I-A)

or a pharmaceutically acceptable salt thereof;
(6) the pharmaceutical composition described in (1), wherein the reversible BTK inhibitor is a triazine derivative (II) shown below,

[chem 5]

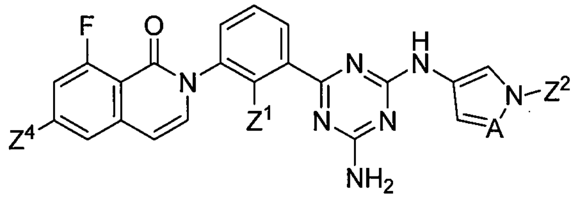

(II)

wherein $Z^1$ represents an optionally substituted lower alkyl group,

$Z^2$ represents a hydrogen atom or a optionally substituted lower alkyl group,
A represents a nitrogen atom or C-$Z^3$,
$Z^3$ represents a hydrogen atom, a cyano group, an optionally substituted acyl group, an optionally substituted sulfonyl group, or an optionally substituted carbamoyl group,
$Z^4$ represents an optionally substituted lower alkyl group, an optionally substituted cycloalkyl group,
or a pharmaceutically acceptable salt thereof;

(7) the pharmaceutical composition described in (6), wherein the reversible BTK inhibitor is a triazine derivative in which $Z^1$ is a hydroxymethyl group;
or a pharmaceutically acceptable salt thereof;
(8) the pharmaceutical composition described in (6), wherein the reversible BTK inhibitor is a triazine derivative having a structure of Compound (II-A);
Compound (II-A): 2-(3-{4-Amino-6-[(1-methyl-1H-pyrazol-4-yl)amino]-1,3,5-triazin-2-yl}-2-(hydroxymethyl)phenyl)-6-cyclopropyl-8-fluoroisoquinolin-1(2H)-one

[chem 6]

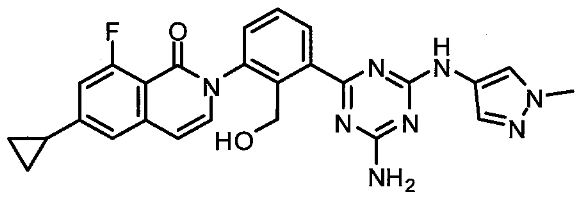

(II-A)

or a pharmaceutically acceptable salt thereof;
(9) the pharmaceutical composition described in (1) to (8), wherein said BCL-2 inhibitor is venetoclax, navitoclax, obatoclax, obatoclax mesylate, sabutoclax,
APG-1252, AZD-0466, APG-2575, ABBV-167, S-65487 or S-55746;
(10) the pharmaceutical composition described in (9), wherein said BCL-2 inhibitor is venetoclax;
(11) the pharmaceutical composition described in (9), wherein said BCL-2 inhibitor is navitoclax;
(12) the pharmaceutical composition described in (9), wherein said BCL-2 inhibitor is S-55746;
(13) the pharmaceutical composition described in (5), wherein said BCL-2 inhibitor is venetoclax;
(14) the pharmaceutical composition described in (5), wherein said BCL-2 inhibitor is navitoclax;
(15) the pharmaceutical composition described in (5), wherein said BCL-2 inhibitor is S-55746;
(16) the pharmaceutical composition described in (8), wherein said BCL-2 inhibitor is venetoclax;
(17) the pharmaceutical composition described in (8), wherein said BCL-2 inhibitor is navitoclax;
(18) the pharmaceutical composition described in (8), wherein said BCL-2 inhibitor is S-55746;
(19) Use of a compound (I) and a BCL-2 inhibitor in manufacturing the pharmaceutical composition described in (1); and

(20) Method for treating a cancer characterized in using any of combinations of medicines described in (1) to (18).

EFFECT OF THE INVENTION

The combination of a reversible BTK inhibitor and a BCL-2 inhibitor can result in cell death with higher efficiency than when the BTK inhibitor or the BCL-2 inhibitor each is used alone. In particular, the BTK inhibitor can regulate abnormal proliferation signals of cancer cells to suppress cell proliferation, while the BCL-2 inhibitor can effectively induce cell death by restoring apoptosis-inducing capacity suppressed in cancer cells. Thus, the combination of the BTK inhibitor and the BCL-2 inhibitor can be expected to exhibit synergistic effects as anticancer action and is useful as a prophylactic or therapeutic pharmaceutical (pharmaceutical composition) for cancer and the like.

BRIEF DESCRIPTION OF DRAWINGS

[Figure 1] Figure 1 is a graph of cell proliferation inhibition rates showing interaction by the combination of a compound (I-A) and a BCL-2 inhibitor (venetoclax) on human DLBCL cell lines (OCI-Ly10).
[Figure 2] Figure 2 is a graph of BLISS score showing interaction by the combination of the compound (I-A) and the BCL-2 inhibitor (venetoclax) on human DLBCL cell lines (OCI-Ly10).
[Figure 3] Figure 3 is a graph of isobologram (50% inhibition) showing interaction by the combination of the compound (I-A) and the BCL-2 inhibitor (venetoclax) on human DLBCL cell lines (OCI-Ly10).
[Figure 4] Figure 4 is a Fa-CI plot showing interaction by the combination of the compound (I-A) and the BCL-2 inhibitor (venetoclax) on human DLBCL cell lines (OCI-Ly10).
[Figure 5] Figure 5 is a graph of cell proliferation inhibition rates showing interaction by the combination of the compound (I-A) and the BCL-2 inhibitor (venetoclax) on BTK-C481S-mutant OCI-LylO cells.
[Figure 6] Figure 6 is a graph of BLISS score showing interaction by the combination of the compound (I-A) and the BCL-2 inhibitor (venetoclax) on BTK-C481S-mutant OCI-LylO cells.
[Figure 7] Figure 7 is a graph of isobologram (50% inhibition) showing interaction by the combination of the compound (I-A) and the BCL-2 inhibitor (venetoclax) on BTK-C481S-mutant OCI-LylO cells.
[Figure 8] Figure 8 is a Fa-CI plot showing interaction by the combination of the compound (I-A) and the BCL-2 inhibitor (venetoclax) on BTK-C481S-mutant OCI-LylO cells.

BEST MODE FOR CARRYING OUT THE INVENTION

(1) Reversible BTK Inhibitors

One of embodiments in the reversible BTK inhibitors is an oxoisoquinoline derivative represented by the following formula (I) described in WO2018/097234 (Patent Document 1);

[chem 7]

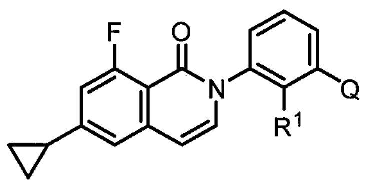

(I)

wherein $R^1$ is an optionally substituted lower alkyl, Q is a structure selected from the following structures (a), (b) and (c);

[chem 8]

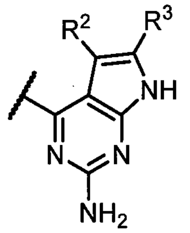 , 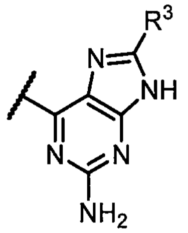 , 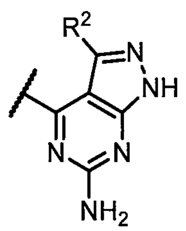

(a) (b) (c)

$R^2$ and $R^3$ are independently a hydrogen atom, an optionally substituted lower alkyl group, an optionally substituted cycloalkyl group, an optionally substituted aryl group, an optionally substituted heteroaryl group or an optionally substituted heterocyclic group,
or a pharmaceutically acceptable salt thereof;

In the specification formula (I) of the present application, a moiety of the lower alkyl group in the "optionally substituted lower alkyl group" may be any of a linear, or branched alkyl group having one to three carbon atoms, and specifically a methyl group, an ethyl group, and an isopropyl group etc. may be exemplified.

A moiety of the cycloalkyl group in the "optionally substituted cycloalkyl group" may be any of cyclic alkyl group having three to six carbon atoms, and specifically a cyclopropyl group, a cyclobutyl group, cyclohexyl group etc. may be exemplified.

A moiety of the aryl group in the "optionally substituted aryl group" may be any of monocyclic or bicyclic aryl group having 6 to 14 carbon atoms, and the bicyclic aryl group may be partially hydrogenated. Specifically, a phenyl group, a naphthyl group, a tetrahydronaphthyl group, an indenyl group etc. may be exemplified.

A moiety of the heteroaryl group in the "optionally substituted heteroaryl group" include a monocyclic aromatic heterocyclic group and a fused aromatic heterocyclic group, and 5- or 6-membered mococyclic aromatic heterocyclic group containing one heteroatom at least selected from a nitrogen atom, a sulfer atom and an oxygen atom as the mococyclic aromatic heterocyclic group. Specifically, pyrrolyl, imidazolyl, pyrazolyl, thienyl, thiazolyl, furanyl, pyridyl, pyrimidyl, pyridazyl etc. may be exemplified, and examples of the fused aromatic heterocyclic group include a fused bicyclic heterocyclic group in which 3- to 8-membered ring is fused containing one heteroatom at least selected from a nitrogen atom, a sulfer atom and an oxygen atom. Specifically tetrahydroisoquinolyl, benzothiophenyl, benzimidazolyl, benzooxazolyl, benzothiazolyl, indolyl, and isoquinolyl may be exemplified.

A moiety of the heterocyclic group in the "optionally substituted hetercyclic group" is a 4- to 6-membered monocyclic saturated heterocyclic group containing one heteroatom at least selected from a nitrogen atom, a sulfer atom and an oxygen atom and may include an unsaturated bond partially in the ring. Specifically, a dihydrothiopyranyl group, 1,1-dioxo-dihydrothiopyranyl group, and tetrahydropyridyl group may be exemplified, and the tetrahydropyridyl group is especially preferrably exemplified.

A substituent of the term of "optionally substituted" in the optionally substituted lower alkyl group, the optionally substituted cycloalkyl group, the optionally substituted aryl group, the optionally substituted heteroaryl group, and the optionally substituted heterocyclic group may be the same or different when the above group have two or more substituents, and the group may be substituted with one, or two or more of any kind of substituent(s) at any position which is chemically allowable.

Examples of the substituent in the optionally substituted lower alkyl group include for example, a halogen atom, a C1-C4 alkoxy group, an amino group optionally substituted with one or two C1-C4 alkyl group, a nitro group, a cyano group, a hydroxy group, a carbamoyl group optionally substituted with one or two C1-C4 alkyl group, a carboxyl group, a formyl group, an acetyl group, a mesyl group, a benzoyl group, a C1-C6 acylamino group, a C1-C6 acyloxy group etc. As the optionally substituted lower alkyl group, a hydroxymethyl group may be exemplified.

Examples of a substituent related to the term of "optionally substituted" in the optionally substituted cycloalkyl group, the optionally substituted aryl group, the optionally substituted heteroaryl group and the optionally substituted heterocyclic group include a halogen atom, an oxygen atom, a C1-C4 alkyl group, a C1-C4 alkoxy group, an amino group optionally substituted with one or two C1-C4 alkyl group, a nitro group, a cyano group, a hydroxy group, a carbamoyl group optionally substituted with one or two C1-C4 alkyl group, a sulfonyl group optionally substituted with a C1-C4 alkyl group, a carboxy group, a formyl group, an acetyl group, a mesyl group, a benzoyl goup, an oxetanyl group, a C1-C6 acylamino group, and a C1-C6 acyloxy group etc.

Isomers may exist in the compound (I) of the present invention, depending on the kind of the substituent. In the present specification, the isomers may be described by a chemical structure of only one form thereof, but the present

invention includes all isomers (geometrical isomer, optical isomer, tautomer, etc.) which can be structurally formed, and also includes isomers alone, or a mixture thereof.

Examples of a pharmaceutically acceptable salt of the compound (I) of the present invention include inorganic acid salts with hydrochloric acid, sulfuric acid, carbonic acid, and phosphoric acid etc.; and organic acid salts with fumaric acid, maleic acid, methanesulfonic acid, and p-toluenesulfonic acid etc.. The present invention also includes ammonium salts, in addition to alkali metal salts with sodium and potassium; alkaline earth metal salts with magnesium and calcium; organic amine salts with triethylamine and ethanolamine; and basic amino acid salts with lysine, arginine, and ornithine etc.

The compound (I) and a pharmaceutically acceptable salt thereof in the present invention can be produced, for example, by methods described in Patent Document 1. When a defined group may be chemically affected under the conditions of an exemplified method in the production method shown below, or is unsuited for use to carry out the method, it is possible to easily produce them by a method which is usually used in organic synthetic chemistry, for example, a method of applying means such as protection or deprotection of a functional group [T. W. Greene, Protective Groups in Organic Synthesis 3rd Edition, John Wiley&Sons, Inc., 1999]. If necessary, the order of a reaction step such as introduction of substituents can also be changed.

Preferably Q is a structure (a) and $R^1$ is a hydroxymethyl group in the compound (I) above, and more preferably, the compound (I) is Compound (I-A): 2-(3-{2-amino-6-[1-(oxetan-3-yl)-1,2,3,6-tetrahydropyridin-4-yl]-7H-pyrrolo[2,3-d]pyrimidin-4-yl}-2=(hydroxymethyl)phenyl)-6-cyclopropyl-8-fluoroisoquinolin-1(2H)-one.

[chem 9]

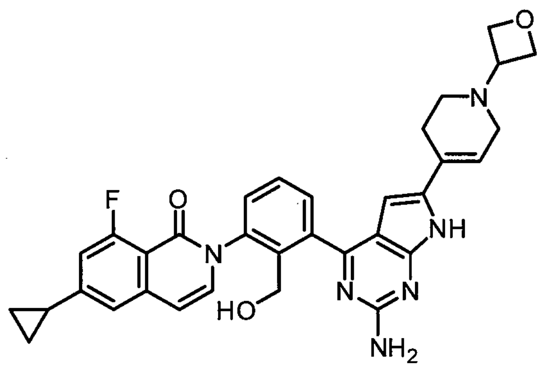

(I-A)

Additionally, Compound (I-A) is a compound of example 23 in the Patent Document 1.

Another embodiment of the reversible BTK inhibitor is a triazine derivative and a pharmaceutically acceptable salt thereof represented by the formula (II) described in WO2015/012149 (Patent Document 2);

[chem 10]

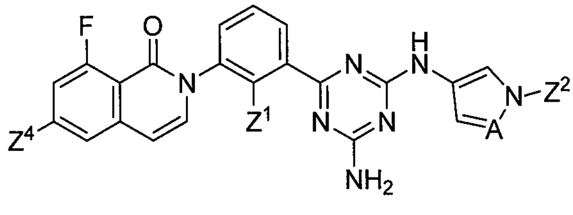

(II)

wherein $Z^1$ represents an optionally substituted lower alkyl group,

$Z^2$ represents a hydrogen atom or a optionally substituted lower alkyl group,
A represents a nitrogen atom or C-$Z^3$,
$Z^3$ represents a hydrogen atom, cyano group, an optionally substituted acyl group, an optionally substituted sulfonyl group, or an optionally substituted carbamoyl group,
$Z^4$ represents an optionally substituted lower alkyl group, an optionally substituted cycloalkyl group;
or a pharmaceutically acceptable salt thereof.

In the compound (II), an lower alkyl group moiety of the optionally substituted lower alkyl group may be any of linear, branched or cyclic alkyl groups having 1 to 3 carbon atoms, and specific examples thereof include a methyl group and an isopropyl group, etc.

A cycloalkyl group moiety of the optionally substituted cycloalkyl group may be cyclic alkyl groups having 3 to 6 carbon atoms, and specific examples thereof include a cyclopropyl group and a cyclobutyl group, etc.

An acyl group moiety of the optionally substituted acyl group may be any of linear, branched or cyclic alkyl groups connected to a carbonyl group, and specific examples of the acyl group moiety of the optionally substituted acyl group include a formyl group, an acetyl group and a propionyl group, a octanoyl group, a dodecanoyl group, a pivaloyl group, a cyclopropylcarbonyl group and a benzoyl group etc.

Examples of the sulfonyl group moiety of the optionally substituted sulfonyl group include a methylsulfonyl group, an ethylsulfonyl group, etc.

Examples of the carbamoyl group moiety of the optionally substituted carbamoyl group include a methylcarbamoyl group, an ethylcarbamoyl group and a dimethylcarbamoyl group, etc.

A substituent of the optionally substituted lower alkyl group, the optionally substituted cycloalkyl group, the optionally substituted acyl group, the optionally substituted sulfonyl group, or the optionally substituted carbamoyl group may be the same or different when the above group have two or more substituents, and the group may be substituted with one, or two or more of any kind of substituent(s) at any position which is chemically allowable. Examples of the substituent include a halogen atom, a substituted or unsubstituted alkyl group, a substituted or unsubstituted alkoxy group, a substituted or unsubstituted amino group, a nitro group, a cyano group, a hydroxy group, a substituted or unsubstituted alkylamino group, a substituted or unsubstituted carbamoyl group, a carboxyl group, a formyl group, an acetyl group, a mesyl group, a benzoyl group, a substituted or unsubstituted acylamino group, and a substituted or unsubstituted acyloxy group, etc.

Isomers may exist in the compound (II) of the present invention, depending on the kind of the substituent. In the present description, the isomers may be described by a chemical structure of only one form thereof, but the present invention includes all isomers (geometrical isomer, optical isomer, tautomer, etc.) which can be structurally formed, and also includes isomers alone, or a mixture thereof.

Examples of the pharmaceutically acceptable salt of the compound (II) of the present invention include inorganic acid salts with hydrochloric acid, sulfuric acid, carbonic acid, and phosphoric acid; and organic acid salts with fumaric acid, maleic acid, methanesulfonic acid, and p-toluenesulfonic acid. The present invention also includes ammonium salts, in addition to alkali metal salts with sodium and potassium; alkaline earth metal salts with magnesium and calcium; organic amine salts with lower alkylamine and lower alcoholamine; and basic amino acid salts with lysine, arginine, and ornithine.

The compound (II) and a pharmaceutically acceptable salt thereof in the present invention can be produced, for example, by methods described in Patent Document 2. When a defined group may be chemically affected under the conditions of an exemplified method in the production method shown below, or is unsuited for use to carry out the method, it is possible to easily produce them by a method which is usually used in organic synthetic chemistry, for example, a method of applying means such as protection or deprotection of a functional group [T. W. Greene, Protective Groups in Organic Synthesis 3rd Edition, John Wiley&Sons, Inc., 1999]. If necessary, the order of a reaction step such as introduction of substituents can also be changed.

Preferably, A is a nitrogen atom and $Z^1$ is a hydroxymethyl group in the compound (II), and more preferably the compound (II) is Compound (II-A): 2-(3-{4-amino-6-[(1-methyl-1H-pyrazol-4-yl)amino]-1,3,5-triazin-2-yl}-2-(hydroxymethyl)phenyl)-6-cyclopropyl-8-fluoroisoquinolin-1(2H)-one

[chem 11]

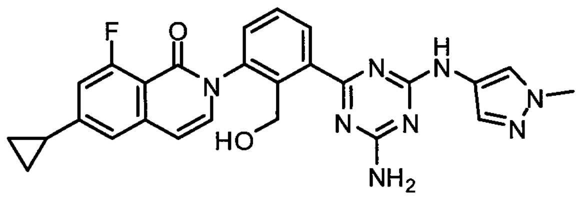

(II-A)

Additionally, Compound (II-A) is a compound of example 1 in the Patent Document 2.

(2) BCL-2 inhibitors

In the present invention, the BCL-2 inhibitor refers to an agent that has an inhibitory action on the physiological function of BCL-2 in a cell. The BCL-2 inhibitor encompasses agents of low molecular weight compounds, polypeptides, proteins, nucleic acids (siRNA, miRNA, aptamers, etc.), other polymeric compounds, or the like, which inhibit the physiological function of BCL-2.

BCL-2 is a member of the BCL-2 family that controls cell death and negatively regulates apoptosis. BCL-2 is also activated in lymphoid B-cell tumors such as follicular lymphoma DLBCL) and CLL as well as in multiple myeloma and T-cell tumors. Since the BCL-2 inhibitor induces apoptosis to these cancer cells to exhibit antitumor effects, in addition to those by the single agent, further antitumor effects can be expected by combining with other agents.

Examples of the BCL-2 inhibitor include venetoclax, navitoclax, obatoclax, obatoclax mesylate, sabutoclax, APG-1252, AZD-0466, APG-2575, ABBV-167, S-65487, and S-55746.

(3) Anticancer agent composition

The anticancer agent composition according to the present invention is a combination agent that appropriately combines a reversible BTK inhibitor with a BCL-2 inhibitor, and encompasses a kit including the reversible BTK inhibitor and the BCL-2 inhibitor. Both inhibitors can be used effectively, together as a mixture, or each separately, for pharmaceuticals, especially for the treatment of tumors. Examples of the tumors include solid tumors such as breast cancer, colorectal cancer, and lung cancer, and hematological cancers such as leukemia, lymphoma, and myeloma.

The anticancer agent composition according to the present invention can be prepared and used in the form of conventional pharmaceutical formulations (pharmaceutical compositions) suitable for oral, parenteral, or topical administration.

Examples of the formulations for oral administration include solid formulations such as tablets, granules, powders, and capsules, and liquid formulations such as syrups. These formulations can be prepared by conventional methods. Solid formulations can be prepared by using conventional pharmaceutical carriers such as lactose, starches, e.g. cornstarch, crystalline cellulose, e.g. microcrystalline cellulose, hydroxypropyl cellulose, calcium carboxymethylcellulose, talc, and magnesium stearate. The capsule can be prepared by encapsulating the granules or powder thus prepared into a capsule. The syrup can be prepared by dissolving or suspending a compound according to the present invention or a pharmaceutically acceptable salt thereof in an aqueous solution containing sucrose, carboxymethylcellulose, or the like.

Examples of the formulation for parenteral administration include an infusion such as an intravenous infusion. Infusion formulations can also be prepared by conventional methods, and can be appropriately incorporated into isotonic agents (e.g., mannitol, sodium chloride, glucose, sorbitol, glycerol, xylitol, fructose, maltose, mannose), stabilizers (e.g., sodium sulfite, albumin), and preservatives (e.g., benzyl alcohol, methyl p-oxybenzoate).

In the present invention, the reversible BTK inhibitor and the BCL-2 inhibitor may be administered concurrently as a formulation of a mixture or as separate formulations. Alternatively, they may be administered in any order sequentially or with appropriate intervals as separate formulations.

The usage and dosage of the anticancer agent composition according to the present invention can vary depending on the reversible BTK inhibitor and the BCL-2 inhibitor to be combined, as well as according to the method of administration (oral, parenteral, or topical administration), the type of disease to which the composition is applied, the severity of the disease, the age and weight of the patient, or the like. Both inhibitors can be typically administered in the range of 1 mg to 1,000 mg per day in adults, and can be administered by oral or parenteral route once or two or three times by divided dosage.

Above all, the anticancer agent composition of the present invention, containing the combination of the compound (I-A), as a reversible BTK inhibitor, and venetoclax, as a BCL-2 inhibitor, can be suitably used for the treatment of tumors.

EXAMPLES

Test Example 1 Cell proliferation suppression test of single agents

(Cell culturing)

Human lymphoma cell line OCI-LylO cells (obtained from University Health Network) were cultured in a 5% $CO_2$ incubator using IMDM medium (Iscove's Modified Dulbecco's Medium, Thermo Fisher Scientific Inc.) containing 20% fetal bovine serum (GE Healthcare) and 1% penicillin streptomycin (NACALAI TESQUE, INC.).

Human lymphoma cell line U2932 cells (obtained from DSMZ) were cultured in a 5% $CO_2$ incubator using RPMI

medium (Roswell Park Memorial Institute medium, Thermo Fisher Scientific Inc.) containing 10% fetal bovine serum (GE Healthcare) and 1% penicillin streptomycin (NACALAI TESQUE, INC.).

(Cell proliferation suppression test)

The OCI-LylO or U2932 cells (20000 cells/well) were seeded into 96-well plates (cell plates), to which the test compound diluted with the medium was added at final concentrations of 0.009 nM to 30000 nM (a final DMSO concentration of 0.3%), and after 96 hours of culture, an Alamar Blue reagent (Thermo Fisher Scientific Inc.) was added. After 3 hours, the fluorescence of excitation wavelength 560 nm/fluorescence wavelength 590 nm was measured and $IC_{50}$ values of inhibitory activity were determined by setting a compound-free and cell-free well as 100% and a compound-free and cell-containing well as 0%. The results are shown in Table 1.

[Table 1]

| Tested Compound | | Inhibitory Activity on cell proliferation $IC_{50}$ (μM) | |
|---|---|---|---|
| | | OCI-LylO | U2932 |
| BTK Inhibitor | (I-A) | 0.002 | 12.5 |
| | (II-A) | 0.042 | 34.7 |
| BCL-2 Inhibitor | venetoclax | 0.01 | 0.72 |
| | S-55746 | 0.069 | 1.35 |
| | obatoclax mesylate | 0.15 | 0.19 |
| | navitoclax | 0.068 | 0.39 |

Example 1 Evaluation of agent combined effect of BTK inhibitor and BCL-2 inhibitor using BLISS score

The BLISS score is one of the criteria used to evaluate synergistic effects in combination of agents (Borisy et al. Proc. Natl. Acad. Sci. USA., 100(13): 7977-7982 (2003); Griner et al. Proc. Natl. Acad. Sci. USA., 111(6): 2349-54 (2014)). This BLISS score was used to analyze the combined effect of a BTK inhibitor and a BCL-2 inhibitor in terms of proliferation inhibition to OCI-LylO cells.

To the cell plates of OCI-LylO cells, a BTK inhibitor (compound (I-A)) (7 doses of 0.03 nM to 30 nM and a DMSO control) and a BCL-2 inhibitor (venetoclax) (7 doses of 0.1 nM to 90 nM and a DMSO control) were added in a combination of 8 × 8 matrices (a final DMSO concentration of 0.6%). After 96 hours of culture, an Alamar Blue reagent was added, and 3 hours later, fluorescence of excitation wavelength 560 nm/fluorescence wavelength 590 nm was measured. Cell proliferation inhibition rate and viability (100 - inhibition rate%) were determined by setting a compound-free and cell-free well as 100% and a compound-free and cell-containing well as 0%.

(Evaluation by BLISS score)

Theoretical BLISS independence (BLISSin) for each well can be calculated from the following formula when agent A and agent B each are thought to have acted as a single agent (i.e., cell proliferation rate when the concentration of either one compound is 0). G is cell viability.

$$BLISS_{in} = G(A) \times G(B)$$

BLISS score for each well can be derived from a difference between the theoretical BLISSin and a measured value of cell proliferation rate. If the BLISS score is a positive number, it is considered that a synergistic effect is present.

$$BLISS\ score = 100 \times (BLISS_{in} - G)$$

The results of the cell proliferation suppression rate in this study are shown in Figure 1 and the BLISS score is shown in Figure 2.

In this study, as shown in Figures 1 and 2, the BLISS score for the combination of the BTK inhibitor (compound

(I-A)) and the BCL-2 inhibitor (venetoclax) showed positive values. Thus, the results of Example 1 indicate that the combination of the BTK inhibitor and the BCL-2 inhibitor has a strong synergistic effect.

Example 2 Evaluation of agent combined effect of BTK inhibitor (compound (I-A)) and BCL-2 inhibitor (venetoclax) by isobologram method

The isobologram method is one approach to evaluate which combined effect, additive, synergistic, or antagonistic action, is exhibited by the two agents used in combination (Chou Cancer Res. 70(2): 440-6 (2010)). This isobologram method was used to analyze the combined effect of a BTK inhibitor and a BCL-2 inhibitor in terms of proliferation inhibition to OCI-LylO cells.

Using a concentration corresponding to the $IC_{50}$ value of the BTK inhibitor or BCL-2 inhibitor as a single agent as a standard based on the results of the cell proliferation suppression test thereof to OCI-LylO cells, DMSO solutions of the respective agents at a concentration of the $IC_{50}$ value $\times$ 10000 were prepared. The two agents were mixed in 8 ratios of 1:0, 1:1, 1:5, 1:10, 10:1, 5:1, 3:1, and 0:1, and the mixture solution thus prepared was added to the cell plates of OCI-LylO cells such that the final concentrations of the mixture solution were the $IC_{50}$ value $\times$ 0.001 to 30 (at a final DMSO concentration of 0.3%). After 96 hours of culture, an Alamar Blue reagent was added, and 3 hours later, the fluorescence of excitation wavelength 560 nm/fluorescence wavelength 590 nm was measured to determine $IC_{50}$ values for each of the mixing ratios.

The concentrations of the BTK inhibitor and the BCL-2 inhibitor showing 50% proliferation inhibitory action were calculated from the $IC_{50}$ value of the single agents and the mixing ratio of the two agents. The results of plotting the concentration of the BCL-2 inhibitor on the vertical axis and the concentration of the BTK inhibitor on the horizontal axis are shown in Figure 3.

(Evaluation by isobologram)

For agents A and B each, the doses at which the agents exhibit a certain action as a single agent are set as $D_A$ and $D_B$. When the dose-response curves for both agents are parallel, if the dose at which an action obtained with combination of both agents is similar to that obtained with the single administration is on the straight line $D_AD_B$, the action is determined to be additive; if the dose is in the lower left of the straight line $D_AD_B$, the action is determined to be synergistic; and if the dose is in the upper right of the straight line $D_AD_B$, the action is determined to be an antagonistic reaction.

In this study, as shown in Figure 3, the concentration at each mixing ratio to show 50% proliferation inhibitory action is in the lower left of the straight line connecting the $IC_{50}$ values of the respective single agents. Thus, the results of Example 2 indicate that the combination of the BTK inhibitor and the BCL-2 inhibitor according to the present invention has a strong synergistic effect.

Example 3 Evaluation of agent combined effect of BTK inhibitor (compound (I-A)) and BCL-2 inhibitor (venetoclax) by Median-effect analysis

Median-effect analysis is a theory advocated by Chou and Talalay, which is one approach to evaluate which combined effect, additive, synergistic, or antagonistic action, is exhibited by the two agents used in combination (Chou Cancer Res., 70(2): 440-6 (2010)). When the agent concentration is set as D, a 50% suppression concentration (median-effect dose) is set as Dm, a rate of suppressed cells is set as Fa (fraction affected), a rate of non-suppressed cells is set as Fu (fraction unaffected), and m is set as a coefficient, the following relationship formula is established:

$$D = Dm(Fa/Fu)^{1/m}$$

A combination index CI is a quantitative index of a combined effect. In the case that action points of effects are different from each other between two agents, when the concentration of agent A when used in combination is set as $(D_{A+B})_A$, the concentration of agent A used singly achieving x% suppression is set as $(Dx)_A$, the concentration of agent B used in combination is set as $(D_{A+B})_B$, and the concentration of agent B used singly achieving x% suppression is set as $(Dx)_B$, CI is given by the following formula:

$$CI = (D_{A+B})_A/(Dx)_A + (D_{A+B})_B/(Dx)_B$$

From the above, CI is expressed as a function of Fa. By plotting Fa on the horizontal axis and CI on the vertical

axis, a correlation diagram called Fa-CI plot is obtained. A CI less than 1 shows a synergistic action, a CI of 1 shows an additive action, and a CI greater than 1 shows an antagonistic action. The experimental data of the isobologram in Example 2 were used to plot Fa-CI of the BTK inhibitor and the BCL-2 inhibitor for OCI-LylO cells (Figure 4).

As shown in Figure 4, the CI was less than 1 with Fa = 0.5 (50% inhibition rate) or more at any mixing ratio, indicating that the combination of the BTK inhibitor and the BCL-2 inhibitor has a strong synergistic effect.

Example 4 Dose-response study of BTK inhibitor in the presence of BCL-2 inhibitor

Changes in $IC_{50}$ values of the BTK inhibitor in the presence of the BCL-2 inhibitor at various concentrations were examined.

From the result obtained for the BCL-2 inhibitor used as a single agent, BCL-2 inhibitor concentrations for three doses were selected for each cancer cell line. The BTK inhibitor was added to cell plates such that the concentrations of the BTK inhibitor ranged from 0.009 nM to 30000 nM (a final DMSO concentration of 0.4%), and $IC_{50}$ values in the presence of the BCL-2 inhibitor at each concentration were determined in the same manner as in Test Example 1 using a DMSO addition group as a control.

The $IC_{50}$ values in the presence of the BCL-2 inhibitor at each concentration are shown in Tables 2 to 9. In this study, as shown in Tables 2 to 9, the $IC_{50}$ value of the BTK inhibitor decreased dependently on the concentration of the BCL-2 inhibitor in both lymphoma cells, OCI-LylO cells and U2932 cells.

[Table 2]

| Con. of venetoclax (nM) | Inhibitory activity on cell proliferation OCI-LylO $IC_{50}$ (μM) | |
|---|---|---|
| | (I-A) | (II-A) |
| 0 | 0.0021 | 0.042 |
| 1 | 0.0015 | 0.038 |
| 3 | 0.0015 | 0.027 |
| 10 | 0.0013 | 0.019 |

[Table 3]

| Con. of S-55746 (nM) | Inhibitory activity on cell proliferation OCI-LylO $IC_{50}$ (μM) | |
|---|---|---|
| | (I-A) | (II-A) |
| 0 | 0.0021 | 0.033 |
| 10 | 0.0013 | 0.029 |
| 30 | 0.0011 | 0.025 |
| 100 | 0.0006 | 0.018 |

[Table 4]

| Con. of obatoclax mesylate (nM) | Inhibitory activity on cell proliferation OCI-LylO $IC_{50}$ (μM) | |
|---|---|---|
| | (I-A) | (II-A) |
| 0 | 0.0012 | 0.038 |
| 50 | 0.0009 | 0.025 |
| 100 | 0.0009 | 0.028 |

(continued)

| Con. of obatoclax mesylate (nM) | Inhibitory activity on cell proliferation OCI-LylO $IC_{50}$ (μM) | |
|---|---|---|
| | (I-A) | (II-A) |
| 200 | 0.0009 | 0.030 |

[Table 5]

| Con. of navitoclax (nM) | Inhibitory activity on cell proliferation OCI-LylO $IC_{50}$ (μM) | |
|---|---|---|
| | (I-A) | (II-A) |
| 0 | 0.0017 | 0.059 |
| 10 | 0.0014 | 0.051 |
| 30 | 0.0011 | 0.042 |
| 100 | 0.0008 | 0.026 |

[Table 6]

| Con. of venetoclax (nM) | Inhibitory activity on cell proliferation U2932 $IC_{50}$ (μM) | |
|---|---|---|
| | (I-A) | (II-A) |
| 0 | 13.6 | >30 |
| 3 | 4.8 | 8.51 |
| 10 | 1.1 | 2.19 |
| 100 | 0.014 | 0.16 |

[Table 7]

| Con. of S-55746 (nM) | Inhibitory activity on cell proliferation U2932 $IC_{50}$ (μM) | |
|---|---|---|
| | (I-A) | (II-A) |
| 0 | >30 | >30 |
| 100 | 3.48 | 5.74 |
| 300 | 0.48 | 0.93 |
| 1000 | 0.022 | 0.10 |

[Table 8]

| Con. of obatoclax mesylate (nM) | Inhibitory activity on cell proliferation U2932 IC$_{50}$ ($\mu$M) | |
|---|---|---|
| | (I-A) | (II-A) |
| 0 | 25.9 | >30 |
| 50 | 19.6 | 28.7 |
| 100 | 13.5 | 25.8 |

[Table 9]

| Con. of navitoclax (nM) | Inhibitory activity on cell proliferation U2932 IC$_{50}$ ($\mu$M) | |
|---|---|---|
| | (I-A) | (II-A) |
| 0 | 28.5 | >30 |
| 30 | 12.0 | 13.4 |
| 100 | 1.95 | 1.36 |
| 300 | 0.023 | 0.10 |

The results of Example 4 indicate that the combination of the BTK inhibitor and the BCL-2 inhibitor according to the present invention has a combined effect not only on certain cancer cells but also on other cancer cells.

Test Example 2 Cell proliferation suppression effect of single agents on BTK-C481S-mutant lymphoma cells

(Production of BTK-C481S-mutant OCI-LylO cells)

BTK-C481S-mutant OCI-LylO cells were produced by point mutation knock-in using the CRISPR-Cas9 system. Cas9 protein (Thermo Fisher Scientific Inc.), guide RNA prepared with GeneArt(TM) Precision gRNA Synthesis Kit (Thermo Fisher Scientific Inc.), and a single-stranded synthetic oligonucleotide to replace 481st cysteine residue of BTK with a serine residue were introduced into OCI-LylO cells by an electroporation method. The resulting modified OCI-LylO cells were cultured in the presence of ibrutinib, and the grown cells were used as BTK-C481S-mutant OCI-LylO cells. Transduction of the mutation was confirmed by genomic DNA and mRNA sequence analysis.

(Cell proliferation suppression test)

In the same manner as in Test Example 1, IC$_{50}$ values for each inhibitor single agent were determined.
The results are shown in Table 10.

[Table 10]

| Tested Compound | | Inhibitory activity on cell proliferation OCI-LylO BTK-C481S IC$_{50}$ ($\mu$M) |
|---|---|---|
| BTK inhibitor | (I-A) | 0.015 |
| | (II-A) | 0.15 |
| BCL-2 inhibitor | venetoclax | 0.02 |
| | S-55746 | 0.12 |
| | obatoclax mesylate | 0.15 |
| | navitoclax | 0.096 |

Example 5 Evaluation of agent combined effect of BTK inhibitor (compound (I-A)) and BCL-2 inhibitor (venetoclax) on BTK-C481S-mutant lymphoma cells using BLISS score

The BLISS score was calculated in the same manner as in Example 1.
The results of the rate of cell proliferation suppression in this study are shown in Figure 5 and the BLISS score is shown in Figure 6.
In BTK-C481-mutant OCI-LylO cells, the BLISS score for the combination of the BTK inhibitor and the BCL-2 inhibitor shows positive values, as in BTK wild-type OCI-LylO cells, indicating that the combination of a BTK inhibitor and a BCL-2 inhibitor according to the present invention has a strong synergistic effect.

Example 6 Evaluation of agent combined effect of BTK inhibitor (compound (I-A)) and BCL-2 inhibitor (venetoclax) on BTK-C481S-mutant lymphoma cells by isobologram method

Isobologram was plotted in the same manner as in Example 2.
The results are shown in Figure 7.
In BTK-C481-mutant OCI-LylO cells, as in BTK wild-type OCI-LylO cells, the concentrations for each mixing ratio to show 50% proliferation inhibitory action were in the lower left of the straight line connecting the $IC_{50}$ values of single agents. Thus, the results of Example 6 indicate that the combination of a BTK inhibitor and a BCL-2 inhibitor according to the present invention has a strong synergistic effect.

Example 7 Evaluation of agent combined effect of BTK inhibitor compound (I-A)) and BCL-2 inhibitor (venetoclax) on BTK-C481S-mutant lymphoma cells by Median-effect analysis

CI values were calculated in the same manner as in Example 3.
The results are shown in Figure 8.
CI was less than 1 with Fa = 0.5 (50% inhibition rate) or more at any mixing ratio, indicating that the combination of a BTK inhibitor and a BCL-2 inhibitor according to the present invention has a strong synergistic effect in BTK-C481-mutant OCI-LylO lymphoma cells, as in BTK wild-type OCI-LylO cells.

Example 8 Dose-response study of BTK inhibitor in the presence of BCL-2 inhibitor using BTK-C481S-mutant lymphoma cells

BTK-C481S-mutant lymphoma cells were used to examine changes in $IC_{50}$ values of the BTK inhibitor in the presence of various concentrations of BCL-2 inhibitor.
From the results obtained for the BCL-2 inhibitor used as a single agent, BCL-2 inhibitor concentrations for three doses were selected for BTK-C481-mutant OCI-LylO cells. The BTK inhibitor was added to cell plates such that the concentration of the BTK inhibitor ranged from 0.009 nM to 30000 nM (a final DMSO concentration of 0.4%), and $IC_{50}$ values in the presence of the BCL-2 inhibitor at each concentration were determined in the same manner as in Example 1 using the DMSO addition group as a control.
The $IC_{50}$ values in the presence of each concentration of the BCL-2 inhibitor are shown in Tables 11 to 14. In this study, as shown in Tables 11 to 14, the $IC_{50}$ value of the BTK inhibitor also decreased in the BTK-C481-mutant OCI-LylO cells dependently on the concentration of the BCL-2 inhibitor.

[Table 11]

| Con. of venetoclax (nM) | Inhibitory activity on cell proliferation OCI-LylO BTK-C481S $IC_{50}$ (μM) | |
|---|---|---|
| | (I-A) | (II-A) |
| 0 | 0.015 | 0.15 |
| 1 | 0.010 | 0.11 |
| 3 | 0.0069 | 0.080 |
| 10 | 0.0048 | 0.053 |

[Table 12]

| Con. of S-55746 (nM) | Inhibitory activity on cell proliferation OCI-LylO BTK-C481S $IC_{50}$ ($\mu$M) | |
|---|---|---|
| | (I-A) | (II-A) |
| 0 | 0.011 | 0.15 |
| 10 | 0.0079 | 0.094 |
| 30 | 0.0052 | 0.069 |
| 100 | 0.0031 | 0.039 |

[Table 13]

| Con. of obatoclax mesylate (nM) | Inhibitory activity on cell proliferation OCI-LylO BTK-C481S $IC_{50}$ ($\mu$M) | |
|---|---|---|
| | (I-A) | (II-A) |
| 0 | 0.016 | 0.30 |
| 50 | 0.0054 | 0.10 |
| 100 | 0.0044 | 0.095 |
| 200 | 0.0040 | 0.061 |

[Table 14]

| Con. of navitoclax (nM) | Inhibitory activity on cell proliferation OCI-LylO BTK-C481S $IC_{50}$ ($\mu$M) | |
|---|---|---|
| | (I-A) | (II-A) |
| 0 | 0.015 | 0.34 |
| 10 | 0.012 | 0.26 |
| 30 | 0.0082 | 0.21 |
| 100 | 0.0041 | 0.11 |

The results of Example 8 indicate that the combination of the BTK inhibitor and the BCL-2 inhibitor according to the present invention has a combined effect not only on BTK wild-type OCI-LylO cells but also on BTK-C481-mutant OCI-LylO cells.

INDUSTRIAL APPLICABILITY

The present invention provides anticancer agent compositions that result in cell death with higher efficiency and with greater selectivity and specificity for a wide range of cancer cells than when the BTK inhibitor or BCL-2 inhibitor each is used alone.

## Claims

1. A Pharmaceutical composition for treating a cancer comprising a reversible BTK inhibitor and a BCL-2 inhibitor.

2. The pharmaceutical composition according to claim 1, wherein the reversible BTK inhibitor is an oxoisoquinoline derivative of the formula (I)

[chem 1]

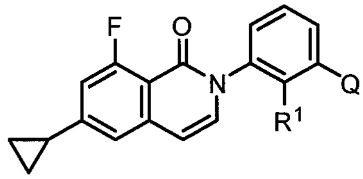

(I)

wherein $R^1$ is an optionally substituted lower alkyl, Q is a structure selected from the following structures (a), (b) and (c);

[chem 2]

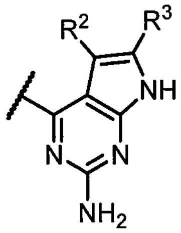 , 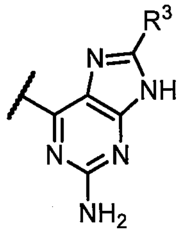 , 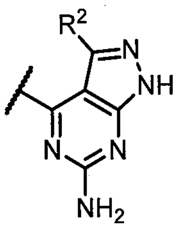

(a) (b) (c)

and $R^2$ and $R^3$ are independently a hydrogen atom, an optionally substituted lower alkyl group, an optionally substituted cycloalkyl group, an optionally substituted aryl group, an optionally substituted heteroaryl group or an optionally substituted heterocyclic group,
or a pharmaceutically acceptable salt thereof.

**3.** The pharmaceutical composition according to claim 2, wherein the reversible BTK inhibitor is an oxoisoquinoline derivative in which Q is the structure (a), and $R^1$ is a hydroxymethyl group,
or a pharmaceutically acceptable salt thereof.

**4.** The pharmaceutical composition according to claim 1, wherein the reversible BTK inhibitor is an oxoisoquinoline derivative represented by the formula (Ia);

[chem 3]

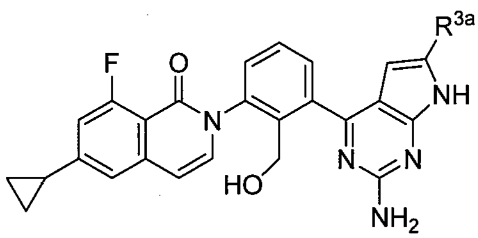

(Ia)

wherein $R^{3a}$ represents an optionally substituted tetrahydropyridyl group,
or a pharmaceutically acceptable salt thereof.

**5.** The pharmaceutical composition according to claim 4, wherein the reversible BTK inhibitor is an oxoisoquinoline derivative having a structure of Compound (I-A);
Compound (I-A): 2-(3-{2-amino-6-[1-(oxetan-3-yl)-1,2,3,6-tetrahydropyridin-4-yl]-7H-pyrrolo[2,3-d]pyrimidin-4-yl}-2-(hydroxymethyl)phenyl)-6-cyclopropyl-8-fluoroisoquinolin-1(2H)-one.

[chem 4]

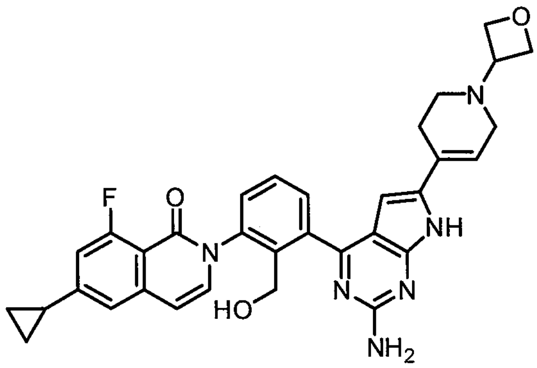

(I-A)

or a pharmaceutically acceptable salt thereof.

6. The pharmaceutical composition according to claim 1, wherein the reversible BTK inhibitor is a triazine derivative (II) shown below,

[chem 5]

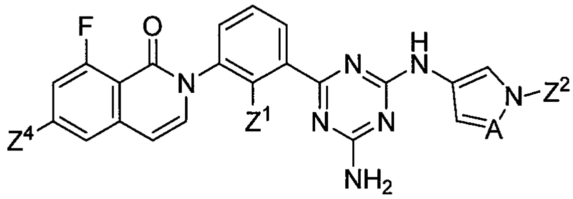

(II)

wherein $Z^1$ represents an optionally substituted lower alkyl group,

$Z^2$ represents a hydrogen atom or a optionally substituted lower alkyl group,
A represents a nitrogen atom or C-$Z^3$,
$Z^3$ represents a hydrogen atom, cyano group, an optionally substituted acyl group, an optionally substituted sulfonyl group, or an optionally substituted carbamoyl group,
$Z^4$ represents an optionally substituted lower alkyl group, an optionally substituted cycloalkyl group,
or a pharmaceutically acceptable salt thereof.

7. The pharmaceutical composition according to claim 6, wherein the reversible BTK inhibitor is a triazine derivative in which $Z^1$ is a hydroxymethyl group,
or a pharmaceutically acceptable salt thereof.

8. The pharmaceutical composition according to claim 6, wherein the reversible BTK inhibitor is a triazine derivative having a structure of Compound (II-A);
Compound (II-A): 2-(3-{4-Amino-6-[(1-methyl-1H-pyrazol-4-yl)amino]-1,3,5-triazin-2-yl}-2-(hydroxymethyl)phenyl)-6-cyclopropyl-8-fluoroisoquinolin-1(2H)-one.

[chem 6]

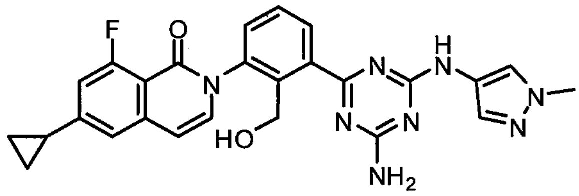

or a pharmaceutically acceptable salt thereof.

9. The pharmaceutical composition according to any of claims 1 to 8, wherein said BCL-2 inhibitor is venetoclax, navitoclax, obatoclax, obatoclax mesylate, sabutoclax, APG-1252, AZD-0466, APG-2575, ABBV-167, S-65487 or S-55746.

10. The pharmaceutical composition according to claim 9, wherein said BCL-2 inhibitor is venetoclax.

11. The pharmaceutical composition according to claim 9, wherein said BCL-2 inhibitor is navitoclax.

12. The pharmaceutical composition according to claim 9, wherein said BCL-2 inhibitor is S-55746;

13. The pharmaceutical composition according to claim 5, wherein said BCL-2 inhibitor is venetoclax.

14. The pharmaceutical composition according to claim 5, wherein said BCL-2 inhibitor is navitoclax.

15. The pharmaceutical composition according to claim 5, wherein said BCL-2 inhibitor is S-55746.

16. The pharmaceutical composition according to claim 8, wherein said BCL-2 inhibitor is venetoclax.

17. The pharmaceutical composition according to claim 8, wherein said BCL-2 inhibitor is navitoclax .

18. The pharmaceutical composition according to claim 8, wherein said BCL-2 inhibitor is S-55746.

19. Use of a compound (I) and a BCL-2 inhibitor in manufacturing the pharmaceutical composition according to claim 1.

20. Method for treating a cancer **characterized in** using a combination of a medicine according to any of claims 1 to 18.

Figure 1

| Venetoclax (nM) \ (I −A) (nM) | 300 | 90 | 30 | 9 | 3 | 0.9 | 0.3 | 0 |
|---|---|---|---|---|---|---|---|---|
| 90 | 99 | 100 | 100 | 98 | 95 | 95 | 93 | 92 |
| 30 | 98 | 98 | 96 | 90 | 82 | 77 | 74 | 74 |
| 9 | 98 | 96 | 89 | 73 | 60 | 51 | 53 | 50 |
| 3 | 96 | 93 | 79 | 56 | 40 | 32 | 31 | 25 |
| 0.9 | 95 | 91 | 68 | 43 | 25 | 20 | 15 | 11 |
| 0.3 | 95 | 90 | 66 | 39 | 19 | 12 | 11 | 5 |
| 0.09 | 93 | 89 | 61 | 26 | 9 | 6 | 3 | 1 |
| 0 | 93 | 87 | 63 | 22 | 4 | -3 | -11 | 0 |

Figure 2

| Venetoclax (nM) \ (I −A) (nM) | 300 | 90 | 30 | 9 | 3 | 0.9 | 0.3 | 0 |
|---|---|---|---|---|---|---|---|---|
| 90 | 0 | **1** | **3** | **4** | **3** | **3** | **2** | 0 |
| 30 | 0 | **2** | **6** | **11** | **7** | **4** | **4** | 0 |
| 9 | **1** | **3** | **8** | **12** | **8** | **3** | **9** | 0 |
| 3 | **1** | **2** | **7** | **15** | **12** | **9** | **14** | 0 |
| 0.9 | **1** | **3** | **1** | **13** | **10** | **12** | **14** | 0 |
| 0.3 | **2** | **2** | **1** | **13** | **10** | **9** | **16** | 0 |
| 0.09 | 0 | **1** | -3 | **4** | **4** | **8** | **13** | 0 |
| 0 | 0 | 0 | 0 | 0 | 0 | 0 | 0 | 0 |

Figure 3

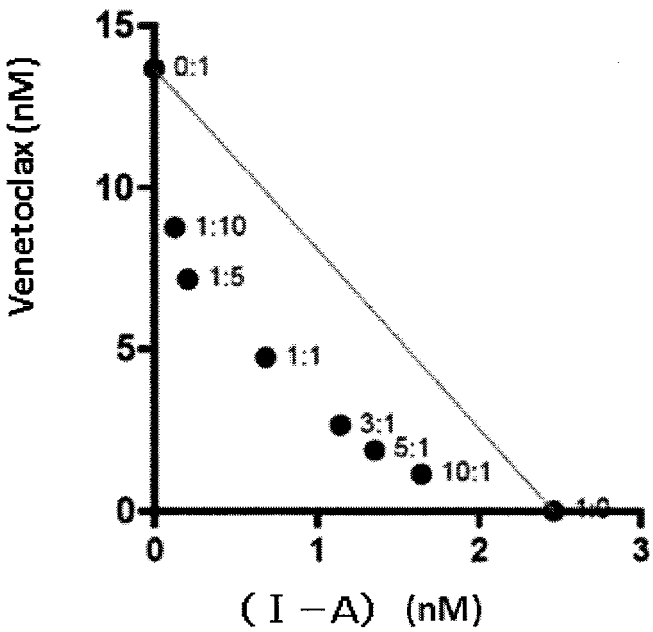


Figure 4

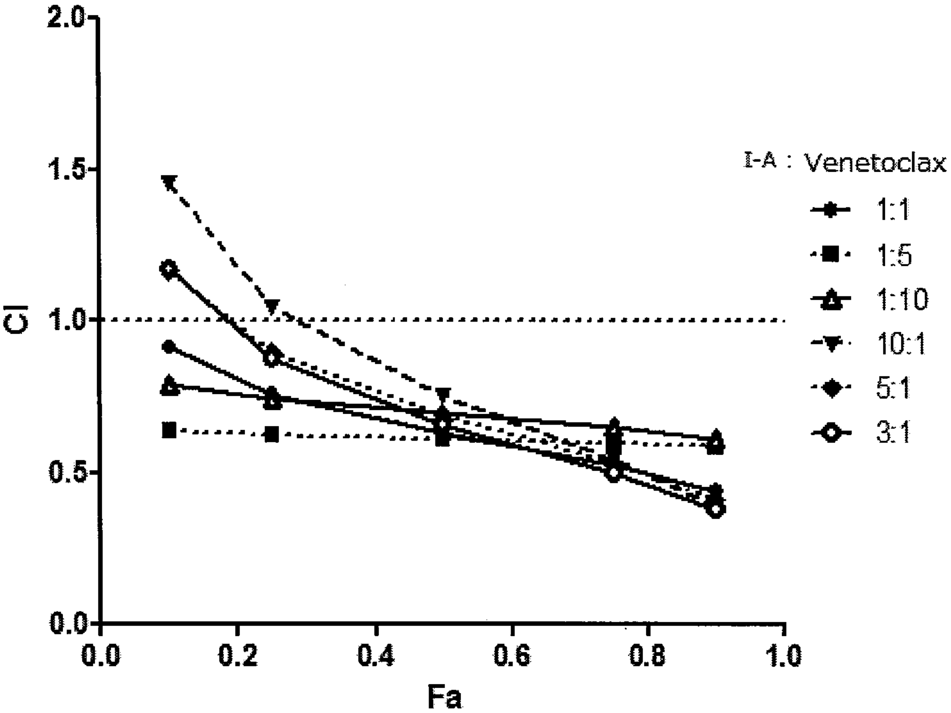

Figure 5

| Venetoclax (nM) \ (I-A) (nM) | 300 | 90 | 30 | 9 | 3 | 0.9 | 0.3 | 0 |
|---|---|---|---|---|---|---|---|---|
| 90 | 99 | 98 | 94 | 88 | 87 | 86 | 85 | 85 |
| 30 | 97 | 90 | 73 | 61 | 61 | 59 | 57 | 54 |
| 9 | 91 | 72 | 49 | 34 | 30 | 28 | 32 | 31 |
| 3 | 81 | 53 | 29 | 17 | 14 | 16 | 14 | 16 |
| 0.9 | 72 | 39 | 18 | 13 | 10 | 9 | 9 | 7 |
| 0.3 | 66 | 28 | 11 | 11 | 5 | 4 | 7 | 4 |
| 0.09 | 61 | 23 | 6 | 1 | 3 | 3 | -2 | 2 |
| 0 | 63 | 18 | 0 | -5 | -8 | -4 | -11 | 0 |

Figure 6

| Venetoclax (nM) \ (I-A) (nM) | 300 | 90 | 30 | 9 | 3 | 0.9 | 0.3 | 0 |
|---|---|---|---|---|---|---|---|---|
| 90 | 4 | 10 | 9 | 4 | 4 | 1 | 1 | 0 |
| 30 | 14 | 28 | 19 | 9 | 11 | 6 | 8 | 0 |
| 9 | 16 | 28 | 18 | 6 | 4 | 0 | 8 | 0 |
| 3 | 12 | 22 | 13 | 5 | 5 | 4 | 7 | 0 |
| 0.9 | 6 | 15 | 11 | 10 | 9 | 6 | 12 | 0 |
| 0.3 | 1 | 7 | 7 | 11 | 9 | 4 | 13 | 0 |
| 0.09 | -2 | 4 | 4 | 4 | 9 | 6 | 7 | 0 |
| 0 | 0 | 0 | 0 | 0 | 0 | 0 | 0 | 0 |

Figure 7

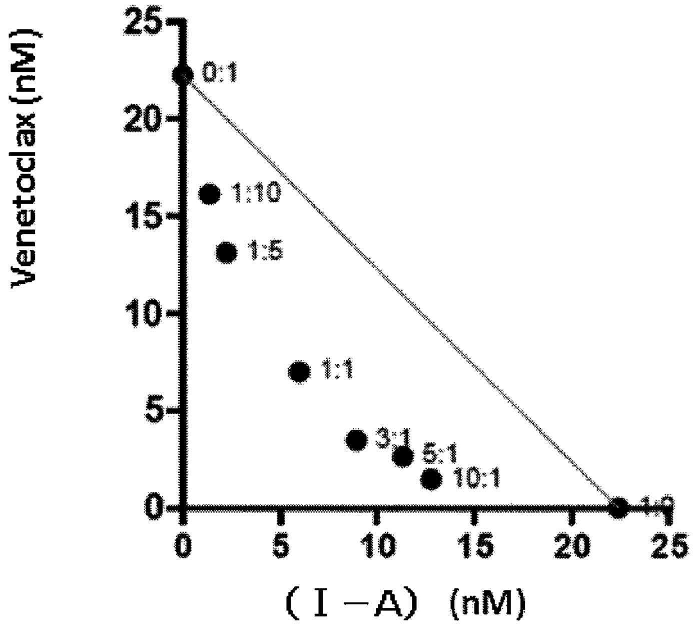


Figure 8

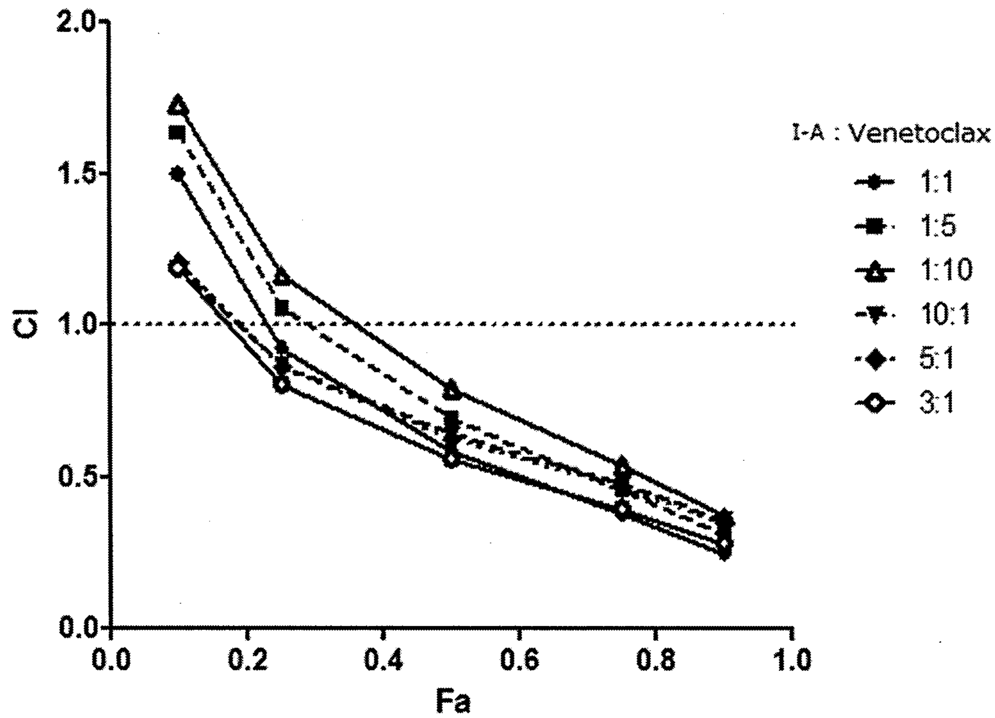

**INTERNATIONAL SEARCH REPORT**

International application No. PCT/JP2021/004083

A. CLASSIFICATION OF SUBJECT MATTER
A61K 45/06(2006.01)i; A61P 35/00(2006.01)i; A61P 35/02(2006.01)i; A61P 43/00(2006.01)i; A61K 31/404(2006.01)i; A61K 31/437(2006.01)i; A61K 31/519(2006.01)i; A61K 31/53(2006.01)i; A61K 31/5377(2006.01)i
FI: A61K45/06; A61P35/00; A61P43/00 121; A61P43/00 111; A61K31/53; A61K31/437; A61K31/5377; A61K31/404; A61P35/02; A61K31/519

According to International Patent Classification (IPC) or to both national classification and IPC

B. FIELDS SEARCHED

Minimum documentation searched (classification system followed by classification symbols)
A61K45/06; A61P35/00; A61P35/02; A61P43/00; A61K31/404; A61K31/437; A61K31/519; A61K31/53; A61K31/5377

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | SINHA, S. et al., "Targeted Axl Inhibition Primes Chronic Lymphocytic Leukemia B Cells to Apoptosis and Show Synergistic/Additive Effects in Combination with BTK inhibitors", Clin. Cancer Res., 2015, vol. 21, no. 9, pp. 2115-2126, doi:10.1158/1078-0432, CCR-14-1892, abstract, page 2120, left column, last paragraph to right column, paragraph [0001], page 2124, left column, paragraph [0004] to right column, fig. 3, 5 | 1, 20<br>1-20 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 March 2021 (11.03.2021) | 30 March 2021 (30.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2021/004083

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | "A Study of Oral LOXO-305 in Patients With Previously Treated CLL/SLL or NHL", ClinicalTrials.gov, NCT0374052, 14 November 2018 [online], [retrieved on 18 March 2021], retrieved from the internet, <URL: https://www.clinicaltrials.gov/ct2/show/study/NCT03740529?term=NCT03740529&draw=2&rank=1>, https://www.clinicaltrials.gov/ct2/show/study/NCT03740529?term=NCT03740529&draw=2&rank=1, in particular, study description | 1, 9, 10, 20<br>1-20 |
| Y | WO 2018/097234 A1 (CARNA BIOSCIENCES, INC.) 31 May 2018 (2018-05-31) claims, paragraphs [0002]-[0004], each example, test examples 1-4 | 1-20 |
| Y | WO 2015/012149 A1 (CARNA BIOSCIENCES, INC.) 29 January 2015 (2015-01-29) claims, paragraph [0009], examples 1, 2 | 1-20 |
| Y | 川畑 亘，ほか，関節リウマチ治療薬を目指したピリミジン骨格を有する非共有結合型選択的 BTK 阻害剤の創製研究，第 35 回メディシナルケミストリーシンポジウム講演要旨集，2017, p. 110, column 1P-048, (KAWAHATA, Wataru et al., "Design and synthesis of a highly selective, non-covalent pyrimidine-based BTK inhibitor for the treatment of rheumatoid arthritis", Lecture abstracts of the 35th Medicinal Chemistry Symposium) | 1-20 |
| Y | JP 2016-521266 A (PHARMACYCLICS LLC) 21 July 2016 (2016-07-21) claims, paragraphs [0028], [0029], [0056], [0187], [0192], [0220], fig. 7-9 | 1-20 |
| Y | JP 2018-522049 A (GILEAD SCIENCES, INC.) 09 August 2018 (2018-08-09) example 1 | 1-20 |
| Y<br>A | US 2018/0221376 A1 (LAM THERAPEUTICS, INC.) 09 August 2018 (2018-08-09) claim 20 | 11-20<br>1-10 |
| Y<br>A | FANG, D. D. et al., "Abstract 2058: BCL-2 selective inhibitor APG-2575 synergizes with BTK inhibitor in preclinical xenograft models of follicular lymphoma and diffuse large B-cell lymphoma", Cancer Research, July 2019, vol. 79, issue 13, supplement, URL: https://cancerres.aacrjournals.org/content/79/13_Supplement/2058, abstract 2058 | 11-20<br>1-10 |
| Y<br>A | US 2019/0248915 A1 (FORTY SEVEN, INC.) 15 August 2019 (2019-08-15) paragraph [0171] | 11-20<br>1-10 |

Form PCT/ISA/210 (continuation of second sheet) (January 2015)

**INTERNATIONAL SEARCH REPORT**

International application No. PCT/JP2021/004083

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | "Study of a New Intravenous Drug, Called S65487, in Patients With Acute Myeloid Leukemia, Non Hodgkin Lymphoma, Multiple Myeloma or Chronic Lymphocytic Leukemia", NCT03755154, ClinicalTrials.gov, 27 November 2018 [online], [retrieved on 11 March 2021], retrieved from the internet, <URL: https://clinicaltrials.gov/ct2/show/NCT03755154?term=S65487&draw=2&rank=2>, https://clinicaltrials.gov/ct2/show/NCT03755154?term=S65487&draw=2&rank=2, in particular, column "official title" | 11-20<br>1-10 |
| A | BOND, D. A. et al., "Targeting BTK in CLL: Beyond Ibrutinib", Current Hematologic Malignancy Reports, 2019, vol. 14, no. 3, pp. 197-205, https://doi.org/10.1007/s11899-019-00512-0, abstract | 1-20 |

Form PCT/ISA/210 (continuation of second sheet) (January 2015)

**INTERNATIONAL SEARCH REPORT**
Information on patent family members

International application No.
PCT/JP2021/004083

| Patent Documents referred in the Report | Publication Date | Patent Family | Publication Date |
|---|---|---|---|
| WO 2018/097234 A1 | 31 May 2018 | US 2019/0359616 A1 claims, paragraphs [0002]-[0011], each example, test examples 1-4<br>EP 3546462 A1<br>CN 109963852 A<br>KR 10-2019-0104142 A | |
| WO 2015/012149 A1 | 29 Jan. 2015 | US 2016/0168122 A1 claims, paragraph [0013], examples 1, 2<br>EP 3026050 A1<br>KR 10-2016-0041946 A<br>CN 105683178 A | |
| JP 2016-521266 A | 21 Jul. 2016 | WO 2014/168975 A1 claims, paragraphs [0065], [0066], [0089], [0225], [0253], fig. 7-9<br>US 2016/0287592 A1<br>EP 2983670 A1<br>CN 105263496 A<br>KR 10-2015-0141971 A | |
| JP 2018-522049 A | 09 Aug. 2018 | WO 2017/023584 A1 example 1<br>US 2018/0207164 A1<br>EP 3331531 A1 | |
| US 2018/0221376 A1 | 09 Aug. 2018 | WO 2018/144680 A1 claim 20<br>EP 3576794 A1<br>KR 10-2019-0110128 A<br>CN 110536702 A<br>JP 2020-505433 A | |
| US 2019/0248915 A1 | 15 Aug. 2019 | WO 2019/157432 A1 paragraph [0171] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018097234 A
- WO 2015012149 A
- WO 2014168975 A


### Non-patent literature cited in the description


- **SATTERTHWAITE,A.B ; WITTE,O.N.** *Immunol.Rev.,* 2000, vol. 175, 120-127
- **KUROSAKI,T.** *Curr.Opin.Immunol.,* 2000, vol. 12, 276-281
- **DAVIS,R.E.** *Nature,* 2010, vol. 463, 88-92
- **ELLMEIER,W.** *FEBS J.,* 2011, vol. 278, 1990-2000
- **HALCOMB,K.E.** *Mol.Immunol.,* 2008, vol. 46 (2), 233-241
- **JANSSON,L.AND ; HOLMDAHL,R.** *Clin.Exp.Immunol.,* 1993, vol. 94, 459-465
- **CHENG,S. et al.** *Leukemia,* 2014, 1-6
- **GRASSILI.E.** *Oncogene,* 2016, vol. 35, 4368-4378
- **CZABOTAR,P.** *Nature Rev. Mol. Cell Biol.,* 2014, vol. 15, 49-63
- **ASHKENAZI,A.** *Nature Rev. Drug Discov.,* 2017, vol. 16, 273-284
- **WILSON W.H.** *Nat.Med.,* 2015, vol. 21 (8), 922-926
- **KUO HP.** *Mol.Cancer Ther.,* 2017, vol. 16 (7), 1246-1256
- **T. W. GREENE.** Protective Groups in Organic Synthesis. John Wiley&Sons, Inc, 1999
- **BORISY et al.** *Proc. Natl. Acad. Sci. USA.,* 2003, vol. 100 (13), 7977-7982
- **GRINER et al.** *Proc. Natl. Acad. Sci. USA.,* 2014, vol. 111 (6), 2349-54
- **CHOU.** *Cancer Res.,* 2010, vol. 70 (2), 440-6